Europäisches Patentamt

European Patent Office

Office européen des brevets

(11) Veröffentlichungsnummer: **0 307 760 B1**

## EUROPÄISCHE PATENTSCHRIFT

(45) Veröffentlichungstag der Patentschrift: **04.11.92**

(21) Anmeldenummer: **88114487.7**

(22) Anmeldetag: **05.09.88**

(51) Int. Cl.5: **G03B 42/02**, G03B 42/04, G01T 1/29

(54) **Röntgenaufnahmekassette für blattförmiges Aufnahmematerial und Verfahren zu deren Verwendung.**

(30) Priorität: **17.09.87 DE 3731204**

(43) Veröffentlichungstag der Anmeldung:
**22.03.89 Patentblatt 89/12**

(45) Bekanntmachung des Hinweises auf die
Patenterteilung:
**04.11.92 Patentblatt 92/45**

(84) Benannte Vertragsstaaten:
**BE DE ES FR GB IT**

(56) Entgegenhaltungen:
**EP-A- 0 077 999**
**EP-A- 0 079 557**
**EP-A- 0 125 412**
**EP-A- 0 231 926**

**PATENT ABSTRACTS OF JAPAN, vol.12, no.
25 (P-659)(2872), 26. Januar 1988 & JP A
62178945**

(73) Patentinhaber: **Agfa-Gevaert AG**

**W-5090 Leverkusen 1(DE)**

(72) Erfinder: **Müller, Jürgen, Dipl.-Ing.**
**Bozzarisstrasse 7**
**W-8000 München 90(DE)**
Erfinder: **Schmidt, Manfred, Dipl.-Ing.**
**Ligusterweg 34**
**W-8011 Kirchheim(DE)**
Erfinder: **Wauer, Dieter, Ing. grad.**
**Flurweg 13**
**W-8028 Taufkirchen(DE)**
Erfinder: **Zehetmaier, Thomas, Dipl.-Ing.**
**Purfinger Strasse 3**
**W-8011 Neufarn(DE)**
Erfinder: **Brys, Georges**
**Lindenlaan 86**
**B-2750 Beverenwaas(BE)**
Erfinder: **Schoeters, Emile**
**Pannenhuisstraat 46**
**B-2500 Lier(BE)**
Erfinder: **Haug, Werner, Dipl.-Ing.**
**Blutenburgstrasse 46**
**W-8000 München 19(DE)**
Erfinder: **Bauer, Walter**
**Heinrich-Wieland-Strasse 178**
**W-8000 München 83(DE)**

EP 0 307 760 B1

**Beschreibung**

Die Erfindung betrifft eine Röntgenaufnahmekassette für ein röntgenstrahlenempfindliches, blattförmiges Aufnahmematerial, vorzugsweise für eine mit einer stimulierbaren Phosphorschicht beschichtete Folie sowie Verfahren zu deren Handhabung.

Eine Kassette der eingangs genannten Art ist bekannt durch die EP 0079557 B1, ein Verfahren zur Verwendung einer derartigen Kassette ist außerdem bekannt durch die EP-A-0 077 999 sowie durch auf dem Markt befindliche Geräte. Die bekannte Kassette mit Aufnahmematerial ist dadurch gekennzeichnet, daß das Aufnahmematerial Identifizierungsmittel aufweist, die auf ihm selbst fest angeordnet sind, und daß die Kassette eine Öffnung aufweist, welche in Übereinstimmung mit den Identifizierungsmitteln angeordnet ist, so daß eine Identifizierung von der Außenseite der Kassette her möglich ist. Dabei können die Identifizierungsmittel durch ein magnetisches Aufzeichnungsmedium gebildet werden, auf das bzw. von dem die Identifizierungsdaten magnetisch aufgezeichnet bzw. gelesen werden können. Als Vorteil hierfür wird angegeben, daß die Identifizierungsdaten direkt auf das Aufnahmematerial aufgebracht sind, also nicht davon getrennt werden und verlorengehen können und daß trotzdem die Identifizierungsdaten von außen aufgebracht bzw. gelesen werden können. Die für die Verwendung einer derartigen Kassette mit phosphorbeschichteter Aufnahmefolie geeigneten, bekannten Verfahren und Vorrichtungen beruhen im wesentlichen darauf, daß bei der Belichtung der in der Kassette befindlichen Folie mit Röntgenstrahlen ein latentes Bild entsteht. In einer Lesestation wird nach dem Entnehmen der Folie aus der Kassette mittels eines Laserstrahlscanners das latente Bild zum Leuchten gebracht (der Phosphor wird stimuliert) und das hierbei ausgesandte Licht in digitale elektrische Bildsignale umgesetzt, die auf einem Bildschirm oder einem Bildschirmaufnahmegerät oder einem computergesteuerten Laserstrahlaufnahmegerät wieder zu einem sichtbaren Bild umgewandelt werden können. Dann wird das Restbild gelöscht und die Folie einem Kassettenbeladegerät zugeführt. Die Identifizierungsdaten auf den Identifizierungsmitteln der Folie müssen dabei, soll ihr Sinn erreicht werden, mit einem hierfür geeigneten gesonderten Lesegerät ausgelesen und in digitale elektrische Identifizierungssignale umgesetzt und den digitalen Bildsignalen zugeführt werden. Andernfalls wären sie zumindest nach dem Löschen des latenten Bildes auf der Folie nutzlos. Die Kassetten, die von der Lesestation aus getrennt von den Folien laufen, enthalten keinerlei Identifizierungsmittel oder Daten, die für sie bzw. ihre Wiederbeladung in einem Kassettenbeladegerät genutzt werden könnten.

Bei der bekannten Kassette für mit einer Schicht aus stimulierbarem Phosphor beschichtete Röntgenaufnahmefolien und dem hierfür bekannten Verfahren ist einerseits von Nachteil, daß die Kassette ein nicht verschließbares Fenster für das folienseitige Identifizierungsmittel aufweisen muß. Wird dabei eine Folie falsch eingelegt, so wird sie an der Stelle des Fensters belichtet und es entsteht hier eine "gelöschte Stelle", die für die Aufnahme nicht brauchbar ist, oder das Identifizierungsmittel wird nicht beschrieben, falls es auf der der Phosphorschicht abgewandten Folienseite liegt. Außerdem ist die Kassettenherstellung aufwendiger als bei einer keine Öffnung aufweisenden Kassette. Schließlich muß trotz des Fensters in der Kassette ein Leser für die Identifizierungsmittel in der Lesestation vorgesehen sein, da anderenfalls die digitalen Bildsignale und die Identifizierungssignale nicht zusammengeführt werden könnten und die Daten auf der Folie trotz des Fensters in der Kassette nutzlos wären. Ein Nachteil des bekannten Verfahrens ist noch darin zu sehen, daß die Folie zwischen dem Entnehmen aus der Kassette in der Lesestation und dem Wiedereinführen in die Kassette im Kassettenbeladegerät, gegen mechanische Einflüsse ungeschützt, ziemliche Wege zurücklegen muß, wodurch eine vorzeitige Abnutzung der Phosphorschicht bedingt sein kann.

Der Erfindung liegt die Aufgabe zugrunde, Kassetten der eingangs genannten Art ohne eine ungeschützte Öffnung auszubilden und trotzdem digitale Identifizierungsdaten in der Kassette zu speichern und ein schonenderes Verfahren für die Behandlung der Folien zu ermöglichen.

Diese Aufgabe wird erfindungsgemäß gelöst durch den Hauptanspruch, insbesondere in Verbindung mit dem Verfahrensanspruch 7. Weitere vorteilhafte Merkmale der Erfindung sind den Unteransprüchen entnehmbar.

Die Erfindung wird anhand von Zeichnungen näher erläutert, wobei auch die Vorteile der erfindungsgemäßen Lösungen eingehend erörtert werden.

Es zeigen

Figur 1    eine Gerätekombination zur Durchführung eines möglichen erfindungsgemäßen Verfahrens unter Verwendung einer erfindungsgemäßen Kassette,

Figur 2    eine Gerätekombination zur Durchführung eines anderen möglichen erfindungsgemäßen Verfahrens unter Verwendung einer erfindungsgemäßen Kassette,

Figur 3    perspektivische Darstellungen erfindungsgemäßer Kassetten unterschiedlicher Formate im Vergleich

Figur 4 eine Ansicht der Vorderseite eines Speichers für die erfindungsgemäßen Kassetten,

Figur 5 eine Ansicht der der jeweiligen Kassettenaußenseite zugewandten Rückseite eines Speichers für die erfindungsgemäßen Kassetten,

Figur 6 eine perspektivische Ansicht einer möglichen, teilweise bekannten Vorrichtung zum Ent- und Beladen von erfindungsgemäßen Kassetten mit einer Leseeinrichtung zum Lesen des Speichers einer Kassette.

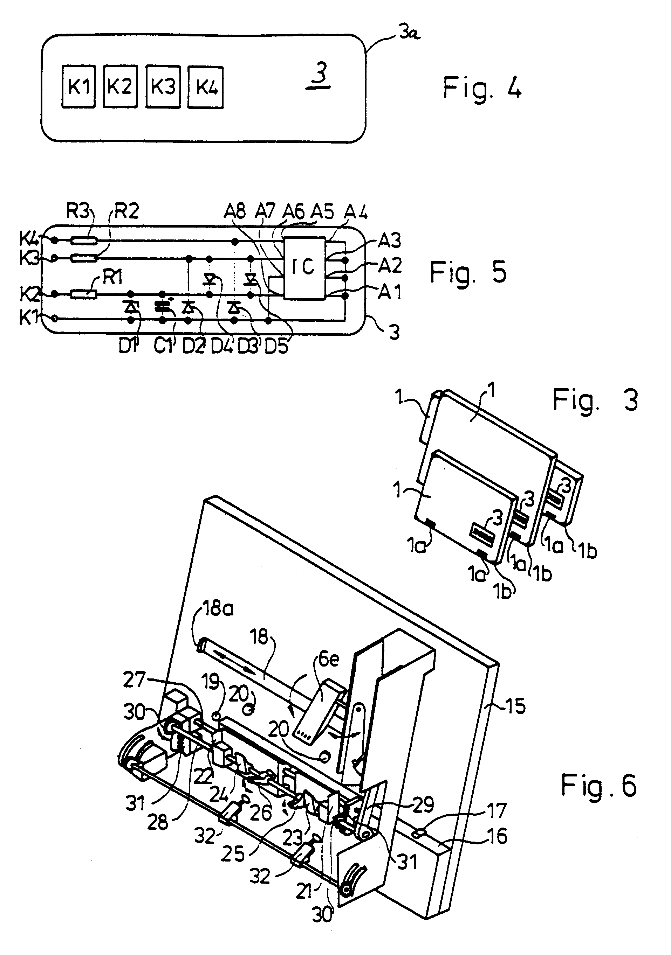

Die Erfindung geht aus von herkömmlichen Röntgenaufnahmekassetten für plane, röntgenstrahlenempfindliche Aufnahmematerialien. Diese Kassetten bestehen aus zwei gegeneinander schwenkbaren Gehäuseteilen, nämlich einem Boden- und einem Deckelteil, wobei gewöhnlich das Bodenteil dem Patienten bzw. den Röntgenstrahlen zugewandt ist. In den Kassetten befinden sich Andruckmittel für das Aufnahmematerial, z.B. eine Schaumstoffplatte. Diese Kassetten sind daher in den Figuren nur schematisch dargestellt und mit 1 beziffert. Diese Kassetten 1 einer jeweils im wesentlichen einheitlichen Bauart mit Mitteln 1a zum Ver- und Entriegeln des Deckelteiles sind in verschiedenen Größen für die vorkommenden Aufnahmeformate vorhanden, wie in Figur 3 anhand von beispielsweise nur drei Kassettenformaten angedeutet ist. Im besonderen soll sich in den Kassetten 1 kein fotografischer Röntgenfilm befinden, sondern eine Aufnahmefolie 2, die mit einer Schicht von stimulierbarem Phosphor beschichtet ist. Auf dieser entsteht bei Belichtung mit Röntgenstrahlen ein latentes Bild, das durch Abtasten mit einem Laserstrahl ausgelesen und in digitale elektrische Bildsignale umgewandelt und abgespeichert wird. Die gespeicherten Bildsignale können dann an einem Bildschirm als sichtbares Bild betrachtet werden. Dementsprechend kann eine sog. Hardcopy auf gewöhnlichem fotografischem Film durch bekannte Verfahren hergestellt werden. Nichtsdestoweniger ist die Benutzung der nachfolgend zu beschreibenden erfindungsgemäßen Kassette 1 für herkömmliche fotografische Röntgenfilme nicht ausgeschlossen.

Vorzugsweise an der Deckelaußenfläche, ggf. aber statt dessen an der Bodenflachseite einer jeden erfindungsgemäßen Kassette 1 in einer bestimmten Lage zu einem Bezugspunkt, die bei allen gleichartigen Kassetten unterschiedlicher Formate gleich ist, ist ein Daten in digitaler Form speichernder, beschreib-, les- und löschbarer Speicher 3 befestigt, der nachfolgend der Einfachheit halber und zur Unterscheidung von anderen Speichern als Kassettenspeicher bezeichnet wird.

Als Bezugspunkt für die Lage des Kassettenspeichers 3 kann eine bestimmte Kassettenecke 1b dienen, die in den Bearbeitungsgeräten für die Kassette 1 bei Kassetten aller Formate an derselben Stelle im jeweiligen Gerät positioniert wird. Diese Lagezuordnung einer bei allen Formaten gleichen Kassettenecke 1b zu der Lage des Kassettenspeichers 3 ist in Figur 3 erkennbar.

Als Speicher kommen entweder Magnetspeicher (Magnetstreifen) oder Halbleiterspeicher in Frage. Magnetspeicher haben den Vorteil, daß sie einfach und preisgünstig sind, aber auch den Nachteil, daß nur geringe Datenmengen speicherbar sind und der Auslesevorgang relativ lange dauert. Deshalb werden im nachfolgenden Halbleiterspeicher als die insgesamt vorteilhaftere Lösung vorausgesetzt. Deren Vorteile bestehen in der schnellen Lesbarkeit, hohen Speicherkapazität, Unempfindlichkeit gegen Magnetfelder und einer geringeren erforderlichen Auslesemechanik. Die Verbindung zwischen Datenspeicher und Ausleseelektronik kann galvanisch oder drahtlos erfolgen. Im Falle der Verwendung eines galvanischen Kontaktes dürfte ein serieller Speicher wegen der geringen Zahl der Anschlüsse vorzuziehen sein.

Anhand der Figuren 4 und 5 wird ein mögliches Ausführungsbeispiel für einen verwendbaren Kassettenspeicher 3 beschrieben. An die verwendete Ausgestaltung des Kassettenspeichers 3 müssen dann auch die Dateneingabe-, Lese- und Löschvorrichtungen in den Geräten, in denen die Kassetten 1 verwendet werden, angepaßt sein. Der Kassettenspeicher weist eine Trägerplatine 3a auf, die auch in den übrigen, stark verkleinerten Figuren erkennbar ist und deshalb pauschal als Kassettenspeicher 3 bezeichnet wird. Auf der der Kassettenflachseite zugewandten Seite der Platine 3a (vgl. Figur 5) ist ein Speicherchip IC gelagert, welches durch zusätzliche passive elektronische Bauteile weitestmöglich auf bekannte Weise vor Zerstörung geschützt ist. So schützt Bauteil D1 in Verbindung mit dem Widerstand R1 gegen eine zu hohe Versorgungsspannung am Kontakt K2. Der Kondensator C1 dient dazu, irgendwelche kurzzeitigen Einbrüche der Versorgungsspannung, z.B. durch schlechten Kontakt, abzufangen. Die Bauteile D2 und D4 schützen in Verbindung mit dem Widerstand R2 den Anschluß A6 des Speicherchips IC. Das Speicherchip IC hat dabei acht Anschlüsse A1 bis A8. Die übrigen Bauteile D3, D5 und R3 haben ähnliche Funktionen. Die vier auf der Oberseite herausgeführten Leitungen K1 bis K4 (vgl. Figur 4) sind an folgende Kontakte der anzuschließenden Geräte zu legen: K1 ist an Masse, K2 an die Versorgungsspannung (+ 5V) zu legen; K3 bildet den Takteingang zur Synchronisierung des Datenflusses und K4 dient dem seriellen Datenein- und -ausgang. Als Beispiel für das Speicherchip IC wird ein galvanisch kontaktiertes EEPROM verwendet.

Die Schaltung ist in SMD-Technik (surface mounted devices) auf die dem Benutzer abgewandte Seite einer doppelseitig kaschierten Leiterplatte aufgebracht. Vier Durchkontaktierungen führen auf vier vergoldete Kontaktflächen K1 bis K4 auf der Oberseite. Mit Hilfe einer Kontaktierungsmechanik wird das Speicher-IC leitend mit der Auslese- und Beschreibelektronik verbunden, die dafür zuständig ist, das EEPROM anhand des richtigen Protokolls anzusprechen, Informationen zu senden und zu empfangen. Zur Übertragung wird ein bekanntes IIC-Bus-Protokoll (IIC-Bus: Inter-IC-Bus) benutzt, mit dem Speicherbausteine verschiedener Hersteller arbeiten.

Das IIC-Bus-Protokoll stellt sicher, daß (auch bei anliegender Versorgungsspannung) eine ungewollte Programmierung des EEPROMs so gut wie ausgeschlossen ist. In Verbindung mit der Tatsache, daß das IC außerhalb der Schreib- Leseeinrichtung stromlos ist, ergibt sich so eine nahezu hundertprozentige Datensicherheit. Eventuelle Fehler durch ausgefallene Bits, die laut Hersteller erst bei über 10.000 Umprogrammierungen zu erwarten sind, lassen sich durch entsprechende Codierung leicht erkennen und beheben. Die Programme in allen Lese- und Schreibeinrichtungen können so geschrieben werden, daß jeder Eintrag automatisch durch geeignete Prüfsummen ergänzt wird und bei jedem Lesevorgang auf Fehlerfreiheit geprüft wird. Je nach Art des Fehlers kann dieser, für den Benutzer unmerklich, korrigiert werden, oder es kann eine Fehlermeldung ausgegeben werden, die z.B. auf eine fehlerhafte Kassette aufmerksam macht. Der Anwender kann dann eine manuelle Fehlerbeseitigung vornehmen oder die Kassette ersetzen. Dabei ist es sinnvoll, daß die Trägerplatine 3a in eine Vertiefung der Kassettenaußenseite eingelassen ist und ihre außenliegende Seite mit der Ebene der Kassettenaußenseite fluchtet oder etwas tiefer liegt, wodurch die Gefahr einer unerwünschten Berührung der Kontakte K1 bis K4 vermindert wird.

Bei dem beschriebenen Ausführungsbeispiel ist es möglich, daß in einem nicht löschbaren, geschützten Datensatz des Kassettenspeichers 3 das jeweilige Kassetten- und damit das darin enthaltene Folienformat z.B. vom Hersteller eingespeichert wird. In einem weiteren Datensatz des Kassettenspeichers 3 kann - gegen unbeabsichtigtes Löschen geschützt - die Art des in der Kassette enthaltenen Aufnahmematerials vom Hersteller oder Benutzer (je nach den vorgesehenen Handhabungsverfahren für die Kassette) eingespeichert werden. Schließlich kann es, in Abhängigkeit von den angewandten Handhabungsverfahren, vorteilhaft sein, wenn im Kassettenspeicher 3 ein veränderbarer Datensatz vorgesehen ist, durch den die Zahl der Belichtungen bzw. Auswertungen oder Löschungen der in der Kassette 1 enthaltenen Folie 2 zählbar ist und der beim Wechsel einer Folie 2 in der Kassette 1 auf Null zurücksetzbar ist.

Die Benutzung der Kassette 1 der beschriebenen Art mit einer phosphorbeschichteten Folie 2 und einem auf einer Kassettenflachseite fest angeordneten, beschreib-, les- und zumindest teilweise löschbaren Speicher 3 läßt sich nach verschiedenen Handhabungsverfahren vornehmen. Diesen Verfahren ist zunächst das bekannte Grundkonzept der Behandlung phosphorbeschichteter Folien 2 gemeinsam, wobei das bei der Belichtung der Folie 2 mit Röntgenstrahlen in einem Röntgengerät 5 entstandene latente Bild in einer Lesestation 6 nach dem Entnehmen der Folie 2 aus der Kassette 1 mittels eines Laserscanners 6a über einen Drehspiegel 6b vorzugsweise bei Weiterbewegung der Folie 2 stimuliert wird, das hierbei ausgesandte Licht über eine Faseroptik und einen Fotomultiplier 6c in digitale elektrische Bildsignale umgesetzt und anschließend das Restbild auf der Folie 2 durch Belichtung mit sichtbarem Licht mittels einer Lampe 6d gelöscht wird. Die digitalen Bildsignale werden in einem Zentralspeicher 7 gespeichert und können von hier aus auf einem Bildschirm 8 wieder in ein sichtbares Bild umgewandelt werden bzw. in einem Bildschirmaufnahmegerät oder einem Laserstrahlaufnahmegerät 9 (beide Geräte sind als Hardcopy-Geräte bekannt) auf fotografisches Blattmaterial in Form eines sichtbaren Bildes aufgenommen werden. Sie können aber auch auf Disketten 10 oder dergl. zur Ablage abgespeichert werden.

Ein neues Verfahrensgrundkonzept speziell für die Handhabung einer erfindungsgemäßen Kassette 1, 2, 3 ist dann so ausgestaltet worden, daß in einer mit einer Bedienungstastatur 4a versehenen Identifikationsstation 4 die für eine spezielle Röntgenaufnahme maßgebenden bildspezifischen Daten (z.B. Patientendaten) in den Kassettenspeicher 3 eingespeichert werden, und zwar entweder unmittelbar vor oder nach der Röntgenaufnahme im Röntgengerät 5, daß in der Lesestation 6 die im Kassettenspeicher 3 gespeicherten bildspezifischen Daten gelesen und zusammen mit den digitalen Bildsignalen der in der Kassette 1 mit Röntgenstrahlen belichteten Folie 2 im Zentralspeicher 7 abgespeichert werden und zusammen mit dem zugehörigen sichtbaren Bild sichtbar bzw. im Hardcopy-Gerät 9 fotografiert werden. Das Löschen der bildspezifischen Daten im Kassettenspeicher 3 nach deren Auswertung in der Lesestation 6 kann entweder an der Leseeinrichtung 6e für den Kassettenspeicher 3 in der Lesestation 6 oder beim Neubeschreiben des Kassettenspeichers 3 in der Identifikationsstation 4 erfolgen. Diese Verfahrensvariante hängt von der Geräteausgestaltung ab.

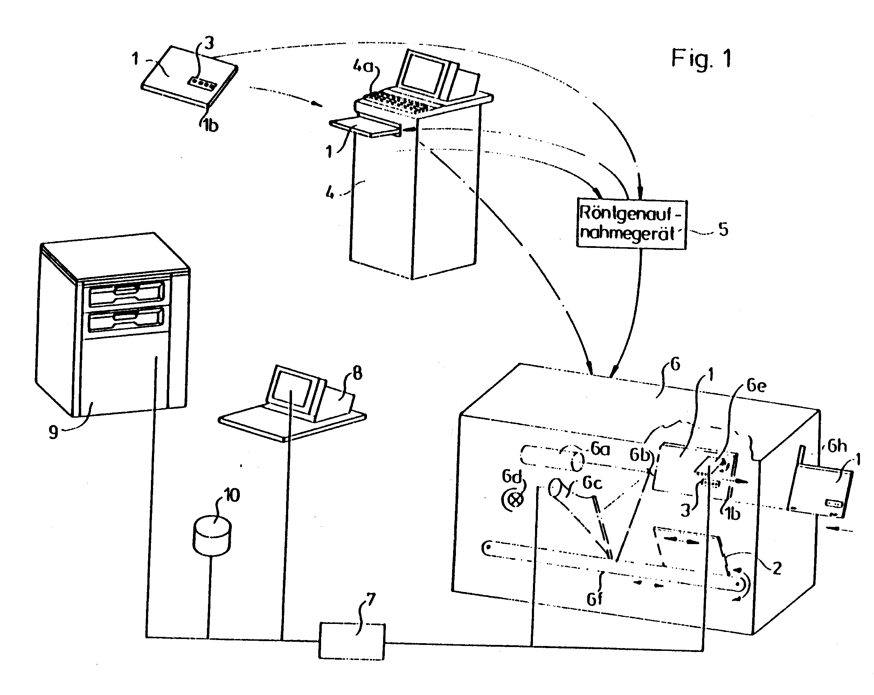

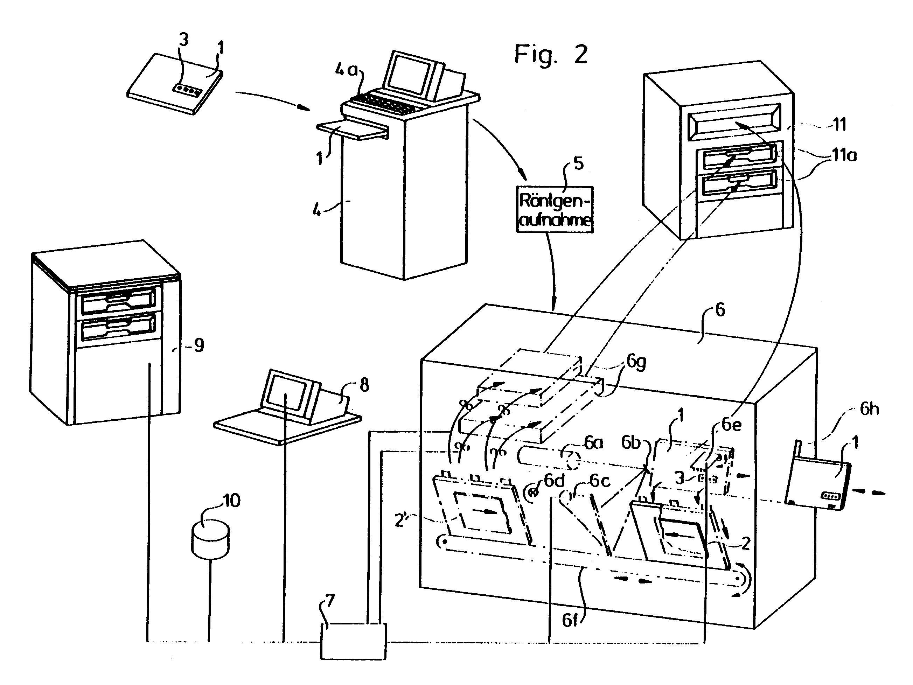

Auf diesem neuen Grundverfahren bauen zwei verschiedene Behandlungsverfahren für erfindungsgemäße Kassetten 1, 2, 3 auf, von denen das anhand von Figur 1 zu Beschreibende das ganz erheblich Vorteilhaftere ist, wie nachfolgend näher erläutert wird. Dabei wird eine belichtete und mit eingespeicherten bildspezifischen Daten versehene Kassette 1 in die Lesestation 6 eingegeben und dort positioniert, worauf dann die bildspezifischen Daten aus dem Kassettenspeicher 3 mittels der Leseeinrichtung 6e gelesen, anschließend die Kassette 1 geöffnet und die Folie 2 der Kassette 1 entnommen werden und schließlich die Folie 2 nach der schon beschriebenen Umsetzung des auf ihr enthaltenen latenten Bildes in die digitalen Bildsignale und deren Einspeicherung in den Zentralspeicher 7 sowie anschließendem Löschen des bildes auf der Folie 2 wieder in dieselbe Kassette 1, aus der sie entnommen worden ist, zurücktransportiert wird. Da es dabei eine Reihe von konstruktiven Möglichkeiten für die Realisierung dieses Verfahrensablaufes gibt und der Transport der Folie 2 durch die Scan- und Löschvorrichtung 6a bis 6d bekannt ist, wurden in den Figuren 1 und 2 die Transportmittel für die Folie 2 in der Lesestation 6 schematisch nur als Transportband angedeutet. Für das Positionieren einer Kassette 1, deren Öffnung, das Entnehmen der Folie 2, das Zurückführen der Folie 2 in dieselbe Kassette 1, das Schließen der Letzteren und Ausgeben aus der Lesestation sind alle bei Kassettenent- und -beladegeräten üblichen Vorrichtungen verwendbar. Eine im wesentlichen bekannte derartige Vorrichtung ist in Figur 6 schematisch gezeigt und wird im Anschluß an die Beschreibung der Verfahren noch kurz erläutert.

Wichtigstes Merkmal dieses Systems aus Kassettenausgestaltung und Kassettenhandhabung ist, daß Folie 2 und Kassette 1 als eine Einheit aufgefaßt werden, die nur zum Auslesen (Scannen) und Digitalisieren des latenten Bildes der Phosphorfolie 2 in der Lesestation 6 getrennt werden. Die Kassetten könnten zwar durch den Benutzer geöffnet werden; dies ist aber nicht vorgesehen, weil dadurch aufgrund des Lichteinfalls die Folie gelöscht würde. Nur falls die Folie einmal ersetzt werden sollte, wird diese Zugehörigkeit Folie/Kassette unterbrochen. Diese Zusammengehörigkeit (zumindest von dem Zeitpunkt der Röntgenaufnahme bis zum digitalisierten Bild) ist Voraussetzung für das hier beschriebene Verfahren der Datenspeicherung auf der Kassette.

Das anhand von Figur 1 beschriebene Verfahren bzw. Kassetten/Kassettenhandhabungs-System hat folgende Vorteile:

1. Der Benutzer hat mit der Folie nichts zu tun. Er bekommt sie nie zu Gesicht, außer bei nach sehr langer Benutzung erforderlichem Folienwechsel.

2. Es entfällt ein gesonderter Kassetten-Beladevorgang samt Kassetten-Beladegerät.

3. Es kann immer davon ausgegangen werden, daß eine Kassette gefüllt ist.

4. Es gibt mehr Möglichkeiten bei der Auswahl des Mediums zur Datenspeicherung, da mehr Platz auf der Kassette vorhanden ist als auf einer Folie. Eine Ausdehnung in die Tiefe ist im Gegensatz zur Folie möglich.

5. Die Kassette kann vollkommen geschlossen ausgelegt und es muß kein Fenster zum Lesen der Daten vorgesehen werden.

6. Über ihre gesamte Lebenszeit kann die Folie und die Kassette von einem veränderbaren Datensatz begleitet werden. Diese bietet die Möglichkeit, das Alter von Kassette und Folie, die Zahl von Belichtungen sowie den Zustand "belichtet/gelöscht" zu erkennen. Die Veranlassung der Fortzählung um "eins" im Kassettenspeicher 3 bei jeder Digitalisierung eines latenten Bildes kann in der Lesestation 6 durch die Leseeinrichtung 6e erfolgen, das auf "Null" Zurücksetzen bei einem Folienwechsel in der Identifikationsstation 4. Zur Einspeicherung des Folienzustandes "belichtet/gelöscht" im Kassettenspeicher 3 müßte noch eine Dateneingabevorrichtung im Röntgengerät 5 und eine entsprechende Löschstelle an der Leseeinrichtung 6e vorgesehen sein. Bei Erreichen einer an die Lebensdauer einer Folie reichenden Aufnahmenanzahl kann in der Lesestation 6 oder der Identifikationsstation 4 ein Warnsignal ausgelöst werden.

7. Patientendaten können vor bzw. bei der Röntgenuntersuchung auf die Kassette geschrieben werden und begleiten ab dann die von der Phosphorfolie registrierten Bildinformationen.

8. Es ist kein weiterer Informationsträger wie z.B. eine Datenkarte nötig.

9. Weiterhin können auch Steuerdaten für die Weiterverarbeitung schon bei der Aufnahme, ohne direkten Kontakt mit der Lesestation eingegeben werden.

10. Bei Vorhandensein unterschiedlicher Folientypen kann auch der Folientyp in den Kassettenspeicher eingespeichert werden.

11. Durch die Zurückführung einer Folie in immer dieselbe Kassette in der Lesestation muß die Folie nur äußerst kurze Wege außerhalb der Kassette zurücklegen, wodurch sie gegen Beschädigungen beim Transport bzw. gegen Abnutzung besser geschützt ist als bei anderen Bearbeitungsverfahren für Kassetten mit phosphorbeschichteten Folien. Dieses Verfahren ist daher auch dann mit Vorteil anwendbar, wenn die Patientendaten nicht auf einem Speicher auf der Kassette gespeichert werden, sondern auf andere Weise den digitalisierten Bildsignalen beigefügt werden, z.B. über einen lesbaren Speicher auf der Folie oder durch direkt den Bildsignalen über eine Tastatur an der Lesestation zugefügte Datensignale.

Das anhand von Figur 2 beschriebene und erkennbare Verfahren zur Bearbeitung erfindungsgemäßer Kassetten 1, 2, 3 unterscheidet sich von dem nach Figur 1 dadurch, daß in der Lesestation 6 die Kassette 1 und die Folie 2 nach dem gemeinsamen Abspeichern der digitalen Bildsignale und der Kassettenspeicher-Daten im Zentralspeicher 7 getrennt weitergeleitet werden, daß die leere Kassette 1 aus der Lesestation 6 ausgegeben wird und einem Kassettenbeladegerät 11 bekannter Bauart zur Neubeladung zuführbar ist, daß in der Lesestation 6 Folienvorratsmagazine 6g für die verschiedenen vorkommenden Folienformate und -sorten angeordnet werden und daß - vom Zentralspeicher 7 aus aufgrund der von der Leseeinrichtung 6e vom Kassettenspeicher 3 abgespeicherten, das Kassetten- und Folienformat und/oder die Foliensorte betreffenden Daten gesteuert - die der Kassette 1 entnommene, gescannte und gelöschte Folie 2' in das ihrem Format und/oder ihrer Foliensorte entsprechende Folienvorratsmagazin 6g mittels hierfür bekannter Transportmittel abgelegt wird. Sind in den Folienvorratsmagazinen 6g jeweils Folienpacks entsprechender Eigenschaft gesammelt, so werden die Folienvorratsmagazine 6g in die entsprechenden Schubladen 11a der Beladevorrichtung 11 für die Kassette 1 eingegeben. Im Beladegerät 11 muß eine der Leseeinrichtung 6e vergleichbare Leseeinrichtung vorgesehen sein, die beim Eingeben einer zu beladenden Kassette 1 von deren Kassettenspeicher 3 Folienformat und -sorte abliest und über eine elektronische Steuervorrichtung in an sich bekannter Weise die Beladung der Kassette aus dem richtigen Folienvorratsmagazin auslöst.

Auch bei diesem Kassettenbearbeitungsverfahren muß die Folie 2 nur einen relativ kleinen Weg völlig ungeschützt zurücklegen. Durch das Umladen in ein Folienvorratsmagazin 6g und den Folientransport im Beladegerät vom Magazin in die Kassette wird aber doch eine erheblich größere Abnutzung verursacht als beim Verfahren nach Figur 1. Daher werden von den für das Verfahren nach Figur 1 aufgezählten Vorteilen 1 bis 11 bei dem Verfahren nach Figur 2 die Vorteile 2, 3, 6 und 11 nicht erreicht.

Für den Transport der Kassette 1 in den verschiedenen Stationen 4, 6, 11 kann jede z.B. von Kassettenent- und Beladegeräten her bekannte Transportvorrichtung verwendet werden, z.B. Reibrollen, Transportwalzen, Transportbänder oder Schieber mit Mitnehmern. Ebenso können die Folien 2 in der Lesestation 6 auf irgendeine, z.B. bei Kassettenent- und Beladegeräten bekannte Weise befördert werden, z.B. durch Sauger oder Transportwalzenpaare (wie dies in Figur 2 zum Transport der Folien aus der Lage 2' in die einzelnen Folienvorratsmagazine angedeutet ist). Das Positionieren der Kassetten zum Auslesen des Kassettenspeichers 3 und zur Entnahme der belichteten Folie sowie das Zurückführen derselben in die Kassette in der Lesestation 6 gemäß Figur 1 oder das Positionieren und Wiederbeladen einer leeren Kassette in einem Beladegerät 11 kann durch jede hierfür von Kassettenent- und Beladegeräten her bekannte Vorrichtung erfolgen, z.B. durch die Ent- und Beladevorrichtung nach der DE 35 44 719 C1. Die dort gezeigte Vorrichtung ist nur der Vollständigkeit halber in etwas abgewandelter, an die Lesestation 6 nach Figur 1 angepaßter Form in Figur 6 als ein mögliches Ausführungsbeispiel gezeigt und wird noch kurz beschrieben. Dabei liegt seitlich hinter dem Kassetteneinführschlitz 6h der Lesestation 6 eine Lager- und Führungsplatte 15. Mit der unteren Begrenzung des vertikalen Einführschlitzes 6h fluchtet eine Führungs- und Auflagebahn 16 für die Kassetten mit einer antreibbaren Reibrolle 17. Ist eine Kassette 1 in den Einführschlitz 6h eingegeben und bis zur Reibrolle 17 geschoben worden, so wird sie von dieser erfaßt und bis zu einem Transportband 18 mit Mitnehmern 18a, von denen nur einer sichtbar ist, geschoben. Nun befördert das Transportband 18 die Kassette bis zur Positioniervorrichtung. Erreicht dabei die vordere Kassettenkante die Lichtschranke 19, so wird der Kassettentransport gestoppt. Mehrere gleichartige Stößel 20, die nur wenig über der Bahn 16 von hinten an der Kassette angreifen, schieben sie von hinten nach vorn in den Bereich der Positionierungsmittel. Diese bestehen aus einem festen Anschlagwinkel 21 und einem mit diesem fluchtenden, verschiebbaren Anschlagwinkel 22. Zwischen beiden sind dreh- und verschiebbare Riegel 23, 24 zum Entriegeln des Kassettendeckels und drehbare Hebel 25, 26 zum wieder Schließen des Kassettendeckels angeordnet. Der Anschlagwinkel 22 samt dem ihm zugeordneten Riegel 24 und Hebel 26 ist auf einer Stange 27 verschiebbar und zusammen mit Riegel 23 und Hebel 24 auf einer Welle 28 gelagert, die über einen fest damit verbundenen Kurbeltrieb 29 drehbar und über fest mit ihr verbundene Zahnräder 30, die mit Zahnstangenstücken 31 kämmen, aufwärts und abwärts verschiebbar ist. Beim Eingeben einer Kassette nimmt die Welle 28 ihre obere Stellung ein, in der die durch sie nicht verdrehbaren Winkelstücke 21, 22 etwa in oder ganz geringfügig unter der Ebene der Bahn 16 liegen. Die Kassette wird also durch die Stößel 20 von der Bahn 16 in den Bereich der Winkelstücke 21, 22 geschoben. Dann wird das bewegliche Winkelstück 22 gegen das feste Winkelstück 21 geschoben, so daß die Kassette zwischen beiden Winkelstücken 21, 22 positioniert wird. Nun wird die schwenkbar vor dem Kassettendeckel liegende Leseeinrichtung 6e gegen den Kassettenspeicher 3 geschwenkt, vgl. Figur 1, liest die zu speichernden Daten aus diesem aus und gibt sie - wie beschrieben - an den Zentralspeicher 7. Dann wird die Leseeinrichtung 6e wieder nach vorn und oben geschwenkt. Die Kurbel 29 dreht nun die Welle 28 entgegen dem Uhrzeigersinn. Dabei werden die Kassette und die Riegel und Hebel nach unten bewegt und zugleich Riegel 23, 24 und Hebel 25, 26 so gedreht und erstere auch verschoben, daß die Kassettenverriegelmittel 1a entriegelt werden und der Kassettendeckel entweder unter der Wirkung einer eigenen Feder aufspringt oder durch einen nicht sichtbaren, an der Kurbel 29 angelenkten Öffnungshaken hochgezogen wird. Mittels bekannter Sauger 32 wird nun die Folie der Kassette entnommen und über weitere bekannte Transportmittel dem Laserstrahlscanner 6a zu- bzw. an diesem vorbeigeführt. Nach dem Löschen der Folie wird sie zurück in den Bereich der Sauger 32 transportiert und durch diese zurück in die Kassette gelegt. Nun verläuft der beschriebene Ablauf mittels der Kurbel 29 rückwärts, Kassette und Welle 28 werden nach aufwärts transportiert und die Hebel 25, 26 erfassen den Kassettendeckel und drücken ihn zu. Wenn die wiederbeladene und geschlossene Kassette mit ihrer Unterkante wieder auf die Ebene der Bahn 16 gelangt ist, wird sie von den Stößeln 20 entgegengesetzt wirkenden, nicht gezeigten Stößeln zurück auf die Bahn 16 geschoben und mittels Transportband 18 und deren sichtbarem Mitnehmer 18a sowie durch die Reibrolle 17 aus dem Einführschlitz 6h wieder ausgegeben. Die Vorrichtung nach Figur 6 ist naturgemäß auch für das Verfahren nach Figur 2 verwendbar, jedoch ohne Wiederbeladungsvorgang für die Kassette.

**Patentansprüche**

1. Röntgenaufnahmekassette für ein röntgenstrahlenempfindliches, blattförmiges Aufnahmematerial, vorzugsweise für eine mit einer stimulierbaren Phosphorschicht beschichtete Folie, dadurch gekennzeichnet, daß in einer von einer bestimmten Ecke (1b) der Kassette (1) einen vorgegebenen Abstand aufweisenden Lage, die bei allen gleichartigen Kassetten unterschiedlicher Formate untereinander gleich ist, ein Patientendaten in digitaler Form speichernder, von außen beschreib- , les- und löschbarer Kassettenspeicher (3) in Form eines Halbleiterspeichers (IC) fest aufgebracht ist.

2. Röntgenaufnahmekassette nach Anspruch 1, dadurch gekennzeichnet, daß als Kassettenspeicher (3) ein Halbleiterspeicher mit galvanischer Datenübertragung durch galvanische Kontaktelemente vorgesehen ist und daß der Halbleiterspeicher (IC) geschützt auf einer, einer Kassettenaußenseite zugewandten Seite einer Trägerplatine (3a) und die mit dem Halbleiterspeicher verbundenen galvanischen Kontaktelemente (K1 bis K4) auf der anderen, außen liegenden Seite der Trägerplatine (3a) angeordnet sind.

3. Röntgenaufnahmekassette nach Anspruch 2, dadurch gekennzeichnet, daß die Trägerplatine (3a) in eine Vertiefung der Kassettenaußenseite eingelassen ist und ihre außen liegende Seite mit der Ebene der Kassettenaußenseite fluchtet oder etwas tiefer liegt.

4. Röntgenaufnahmekassette nach einem der vorhergehenden Ansprüche, dadurch gekennzeichnet, daß in einem nicht löschbaren Datenbereich des Kassettenspeichers (3) das jeweilige Format der Kassetten- und damit das verwendbare Format des Aufnahmematerials einspeicherbar ist.

5. Röntgenaufnahmekassette nach einem der vorhergehenden Ansprüche, dadurch gekennzeichnet, daß in einem nicht löschbaren Datenbereich des Kassettenspeichers (3) die Art des Aufnahmematerials einspeicherbar ist.

6. Röntgenaufnahmekassette für eine phosphorbeschichtete Folie nach einem der vorhergehenden Ansprüche, dadurch gekennzeichnet, daß im Kassettenspeicher (3) ein veränderbarer Datenbereich vorgesehen ist, durch den die Zahl der Belichtungen bzw. Auswertungen bzw. Löschungen der Folie (2) zählbar ist und der beim Wechsel einer Folie (2) auf Null zurücksetzbar ist.

7. Verfahren zur Verwendung einer Kassette (1) mit einer mit stimulierbarem Phosphor beschichteten Folie und einem als Halbleiterspeicher (IC) ausgebildeten Kassettenspeicher (3) nach einem der vorhergehenden Ansprüche, wobei das bei der Belichtung der Folie mit Röntgenstrahlen entstandene Latentbild in einer Lesestation (6) nach dem Entnehmen der Folie aus der Kassette mittels eines Laserstrahlscanners stimuliert wird, das hierbei ausgesandte Licht in digitale elektrische Bildsignale umgesetzt, anschließend die Folie durch Beleuchtung mit hierfür geeignetem, keine Röntgenstrahlen enthaltendem Licht gelöscht wird, die digitalen Bildsignale in einem Zentralspeicher (7) abgespeichert werden und auf einem Bildschirm (8) zu einem sichtbaren Bild umgewandelt und/oder in einem Bildschirmaufnahmegerät oder einem vom Zentralspeicher (7) gesteuerten Laserstrahlaufnahmegerät (9), beide Hardcopy-Geräte genannt, zu einem sichtbaren Bild umgewandelt und auf fotografischen Blattfilm aufgenommen werden, dadurch gekennzeichnet, daß in einer mit einer Bedienungstastatur (4a) versehenen Identifikationsstation (4) die für eine Röntgenaufnahme spezifischen Patientendaten in den Kassettenspeicher (3) eingespeichert werden, daß in der Lesestation (6) die im Kassettenspeicher (3) gespeicherten Daten gelesen und zusammen mit den digitalen Bildsignalen im Zentralspeicher (7) abgespeichert werden und zusammen mit dem zugehörigen, sichtbaren Bild auf dem Bildschirm (8) angezeigt oder im Hardcopygerät (9) auf den fotografischen Blattfilm abgebildet werden.

8. Verfahren nach Anspruch 7, dadurch gekennzeichnet, daß ein Löschen der zu verändernden, für eine Röntgenaufnahme spezifisischen Daten (z.B. Patientendaten) entweder nach deren Auslesen in der Lesestation (6) oder bei der Neubeschriftung der zu verändernden Kassettenspeicherdaten in der Identifikationsstation (4) durchgeführt wird.

9. Verfahren nach einem der Ansprüche 7 oder 8, dadurch gekennzeichnet, daß die Zahl in dem zur Zählung der Kassetten- bzw. Folienbelichtungen dienenden, veränderbaren Datenbereich des Kassettenspeichers (3) bei jeder Datenauslesung und -abspeicherung in der Lesestation (6) um eins erhöht wird, so daß dieser Datenbereich die Gesamtzahl der auf der in der Kassette (1) enthaltenen Folie (2) gemachten Aufnahmen enthält.

10. Verfahren nach Anspruch 9, dadurch gekennzeichnet, daß die Gesamtzahl der Belichtungen einer in der Kassette (1) befindlichen Folie (2) in der Identifikationsstation (4) und/oder in der Lesestation (6) -vorzugsweise automatisch- ausgelesen wird und bei Überschreiten einer für gute Aufnahmeergebnisse noch zulässigen Höchstzahl ein Alarmsignal in der jeweiligen Station (4 bzw. 6) ausgelöst wird.

11. Verfahren nach einem der Ansprüche 9 oder 10, dadurch gekennzeichnet, daß beim Einlegen einer neuen Folie (2) in die Kassette (1) der zur Zählung veränderbare Datenbereich -vorzugsweise in der Identifikationsstation (4)- auf Null zurückgesetzt wird.

12. Verfahren nach Anspruch 7, dadurch gekennzeichnet, daß in der Lesestation (6) die Kassette (1) und die Folie (2) nach dem gemeinsamen Abspeichern der digitalen Bildsignale und der Kassettenspeicher-Daten im Zentralspeicher (7) getrennt weitergeleitet werden, daß die leere Kassette (1) aus der Lesestation (6) ausgegeben wird und einem Kassettenbeladegerät (11) zugeführt wird, daß in der Lesestation (6) Folienvorratsmagazine (6g) für die verschiedenen vorkommenden Folienformate und -sorten angeordnet werden und daß - vom Zentralspeicher (7) aus aufgrund der in der Lesestation (6, 6e) vom Kassettenspeicher (3) ausgelesenen, das Kassetten- und Folienformat und/oder die Foliensorte betreffenden Daten gesteuert - die der Kassette 1 entnommene Folie (2') in das ihrem Format und/oder ihrer Foliensorte entsprechende Folienvorratsmagazin (6g) mittels hierfür bekannter Transportmittel abgelegt wird.

13. Verfahren nach Anspruch 12, dadurch gekennzeichnet, daß die verschiedenen, in der Lesestation (6) mit Folienpacks bestückten Folienvorratsmagazine (6g) in bekannter Weise in ein Kassettenbeladegeräte (11) eingesetzt werden, daß eine zu beladende Kassette (1) auf bekannte Weise in dem Kassettenbeladegerät (11) positioniert wird, daß anschließend aus ihrem Kassettenspeicher (3) durch eine Kassettenspeicher-Lesevorrichtung ihre Format- und Foliensortendaten ausgelesen und an eine an sich bekannte Belade-Steuervorrichtung weitergegeben werden, durch die das den Format- und Foliensortendaten entsprechende Folienvorratsmagazin für die Neubeladung der Kassette bereitgestellt wird, und daß schließlich die Kassette mit der ihren Kassettenspeicherdaten entsprechenden Folie beladen, geschlossen und aus dem Beladegerät (11) ausgegeben wird.

**Claims**

1. X-ray film cassette for X-ray-sensitive exposure material in sheet form, preferably for a film coated with a stimulable phosphorus layer, characterized in that an externally writable, readable and erasable cassette memory (3) in the form of a semiconductor memory (IC), which stores patient data in digital form, is permanently mounted in a position, which is at a preset distance from a specific corner (1c) of the cassette (1) and is identical in all identical-type cassettes of differing formats.

2. X-ray film cassette according to claim 1, characterized in that a semiconductor memory with electric data transmission by means of electric contact elements is provided as a cassette memory (3), and that the semiconductor memory (IC) is disposed in a protected manner on one side, facing an outer surface of the cassette, of a mother board (3a) and the electric contact elements (K1 to K4) connected to the semiconductor memory are disposed on the other, outer-lying side of the mother board (3a).

3. X-ray film cassette according to claim 2, characterized in that the mother board (3a) is let into a recess in the outer surface of the cassette and its outer-lying side is flush with, or is slightly recessed relative to the plane of the outer surface of the cassette.

4. X-ray film cassette according to one of the preceding claims, characterized in that the format of the cassette, and hence the format of exposure material to be used, is stored in a non-erasable data storage area of the cassette memory (3).

5. X-ray film cassette according to one of the preceding claims, characterized in that the type of exposure material is stored in a non-erasable data storage area of the cassette memory (3).

6. X-ray film cassette for a phosphorus-coated film according to one of the preceding claims, characterized in that there is provided in the cassette memory (3) a variable data storage area, by means of which the number of exposures or evaluations or erasures of the film (2) may be counted and which may be reset to zero when a film (2) is changed.

7. Process for using a cassette (1) having a film coated with stimulable phosphorus and a cassette memory (3) in the form of a semiconductor memory (IC) according to one of the preceding claims, wherein the latent image produced upon exposure of the film to X-rays is stimulated in a reading station (6), after removal of the film from the cassette, by means of a laser beam scanner, the light emitted during said process is converted into digital electric image signals, the film is then erased by exposing it to suitable light for said purpose containing no X-rays, the digital image signals are saved in a main memory (7) and are converted on a display screen (8) into a visual image and/or are converted in a video recorder or a laser beam recorder (9) controlled by the main memory (7), both referred to as hard copy appliances, into a visual image and recorded onto photographic sheet film, characterized in that, in an identification station (4) provided with an operator's keyboard (4a), the patient data specific to an X-ray exposure are stored in the cassette memory (3), that in the reading station (6) the data stored in the cassette memory (3) are read and, together with the digital image signals, saved in the main memory (7) and, together with the associated visual image, are displayed on the display screen (8) or are imaged in the hard copy appliance (9) onto the photographic sheet film.

8. Process according to claim 7, characterized in that erasure of the data (e.g. patient data) specific to an X-ray exposure which are to be altered is effected either after they have been read out in the reading station (6) or when the cassette memory data to be altered are relabelled in the identification station (4).

9. Process according to one of claims 7 or 8, characterized in that the number in the variable data storage area of the cassette memory (3) used to count the cassette or film exposures is increased by one each time data are read out and saved in the reading station (6) so that said data storage area contains the total number of exposures of the film (2) contained in the cassette (1).

10. Process according to claim 9, characterized in that the total number of exposures of a film (2) contained in the cassette (1) is read out - preferably automatically - in the identification station (4) and/or the reading station (6) and, when a maximum number which is still acceptable for good exposure results is exceeded, an alarm signal is triggered in the station in question (4 and/or 6).

11. Process according to one of claims 9 or 10, characterized in that upon insertion of a new film (2) into the cassette (1) the variable data storage area for counting is reset - preferably in the identification station (4) - to zero.

12. Process according to claim 7, characterized in that in the reading station (6) the cassette (1) and the film (2), after joint saving of the digital image signals and the cassette memory data in the main memory (7), are separately routed, that the empty cassette (1) is dispensed from the reading station (6) and fed to a cassette loading unit (11), that film supply magazines (6g) for the various film formats and types are disposed in the reading station (6), and that - controlled from the main memory (7) on the basis of the data relating to the cassette and film format and/or the film type which are read out of the cassette memory (3) in the reading station (6, 6e) - the film (2') removed from the cassette 1 is deposited by means of known transport means for said purpose into the film supply magazine (6g) corresponding to its format and/or film type.

13. Process according to claim 12, characterized in that the various film supply magazines (6g) filled in the reading station (6) with film packs are in a known manner inserted into a cassette loading unit (11), that a cassette (1) to be loaded is positioned in a known manner in the cassette loading unit (11), that its format and film type data are then read out of its cassette memory (3) by a cassette memory reading device and transferred to a known loading control device, by means of which the film supply magazine corresponding to the format and film type data is made available for reloading of the cassette, and that finally the cassette, having been loaded with the film corresponding to its cassette memory data, is closed and dispensed out of the loading unit (11).

**Revendications**

1. Cassette radiographique pour un matériau d'enregistrement en feuille sensible aux rayons X, de préférence, pour une feuille enduite d'une couche de phosphore apte à être stimulé, caractérisée en ce que, à un endroit présentant une distance prédéfinie par rapport à un coin déterminé (1b) de la cassette (1), cet endroit étant réciproquement identique dans toutes les cassettes de même type à formats différents, est appliquée à demeure une mémoire de cassette (3) sous forme d'une mémoire à semi-conducteurs (IC) mémorisant des données relatives à un patient sous forme numérique et sur laquelle on peut écrire, lire et effacer de l'extérieur.

2. Cassette radiographique selon la revendication 1, caractérisée en ce que, comme mémoire de cassette (3), est prévue une mémoire à semi-conducteurs à transmission galvanique de données à l'intervention d'éléments galvaniques de contact et en ce que la mémoire à semi-conducteurs (IC) est disposée de manière protégée sur un côté d'une platine de support (3a) tournée vers un côté extérieur de la cassette, et les éléments galvaniques de contact (K1 à K4) reliés à la mémoire à semi-conducteurs sont disposés sur l'autre côté de la platine de support (3a) situé à l'extérieur.

3. Cassette radiographique selon la revendication 2, caractérisée en ce que la platine de support (3a) est encastrée dans un renfoncement du côté externe de la cassette, son côté situé à l'extérieur se trouvant en alignement avec le plan du côté externe de la cassette ou étant disposé un peu plus en profondeur.

4. Cassette radiographique selon l'une quelconque des revendications précédentes, caractérisée en ce que, dans une zone ineffaçable de la mémoire de cassette (3) réservée à des données, on peut mémoriser le format correspondant des cassettes et partant, le format à utiliser quant au matériau d'enregistrement.

5. Cassette radiographique selon l'une quelconque des revendications précédentes, caractérisée en ce que, dans une zone ineffaçable de la mémoire de cassette (3) réservée aux données, on peut mémoriser le type de matériau d'enregistrement.

6. Cassette radiographique pour une feuille enduite de phosphore selon l'une quelconque des revendications précédentes, caractérisée en ce qu'on prévoit une zone modifiable de la mémoire de cassette (3) réservée aux données, par laquelle on peut compter le nombre des expositions ou des évaluations ou encore des effacements de la feuille (2) et que l'on peut remettre à zéro lorsqu'on remplace la feuille (2).

7. Procédé pour l'utilisation d'une cassette (1) munie d'une feuille enduite de phosphore apte à être stimulé et d'une mémoire de cassette (3) réalisée sous forme d'une mémoire à semi-conducteurs (IC) selon l'une quelconque des revendications précédentes, dans lequel on stimule, au moyen d'un scanner à rayons laser, l'image latente que l'on obtient lors de l'exposition de la feuille aux rayons X dans un poste de lecture (6) après avoir retiré la feuille hors de la cassette, on transforme en signaux vidéo électriques numériques la lumière émise en l'occurrence, on efface ensuite la feuille par éclairage avec une lumière appropriée à cet effet, qui ne contient pas de rayon X, on mémorise les signaux vidéo numériques dans une mémoire centrale (7) et on les transforme sur un écran (8) en une image visible et/ou on les transforme en une image visible dans un appareil d'enregistrement à écran ou dans un appareil d'enregistrement à rayon laser (9) commandé par la mémoire centrale (7), tous deux appelés appareils de recopie imprimée, et on les enregistre sur une pellicule photographique, caractérisé en ce qu'on mémorise dans la mémoire de cassette (3), dans un poste d'identification (4) équipé d'un clavier de commande (4a), les données relatives à un patient spécifiques pour radiographie, en ce qu'on lit dans le poste de lecture (6) les données mémorisées dans la mémoire de cassette (3) et on les mémorise conjointement avec les signaux vidéo numériques dans la mémoire centrale (7) et on les affiche sur l'écran (8), conjointement avec l'image visible correspondante, ou bien on les représente sur la pellicule photographique dans l'appareil de recopie imprimée (9).

8.   Procédé selon la revendication 7, caractérisé en ce qu'on effectue un effacement des données à modifier spécifiques pour une radiographie (par exemple, des données relatives à un patient), soit après leur déchiffrage dans le poste de lecture (6), soit lors de la réinscription des données de la mémoire de cassette à modifier, dans le poste d'identification (4).

9.   Procédé selon l'une quelconque des revendications 7 ou 8, caractérisé en ce qu'on élève de un à chaque déchiffrage et mémorisation de données au poste de lecture (6), le chiffre que l'on trouve dans la zone modifiable de la mémoire de cassette (3) réservée aux données servant à compter les expositions des cassettes ou des feuilles, de telle sorte que cette zone réservée aux données contienne le nombre total des enregistrements réalisés sur la feuille (2) contenue dans la cassette (1).

10.   Procédé selon la revendication 9, caractérisé en ce qu'on lit - de préférence de manière automatique - le nombre total des expositions d'une feuille (2) se trouvant dans la cassette (1) au poste d'identification (4) et/ou au poste de lecture (6), un signal d'alarme se déclenchant dans chacun des postes (4 ou 6) lorsqu'on dépasse un nombre maximal encore acceptable pour obtenir de bons résultats quant à l'enregistrement.

11.   Procédé selon l'une quelconque des revendications 9 ou 10, caractérisé en ce que, lors de l'introduction d'une nouvelle feuille (2) dans la cassette (1), on remet à zéro - de préférence au poste d'identification (4) - la zone modifiable réservée aux données à des fins de comptage.

12.   Procédé selon la revendication 7, caractérisé en ce que, au poste de lecture (6), on transmet de manière séparée dans la mémoire centrale (7), la cassette (1) et la feuille (2) après la mémorisation commune des signaux vidéo numériques, ainsi que les données provenant de la mémoire de cassette, en ce qu'on retire la cassette vide (1) hors du poste de lecture (6) et on l'achemine à un appareil de chargement de cassettes (11), en ce que, dans le poste de lecture (6), on dispose des magasins d'alimentation en feuilles (6g) pour les divers formats et types de feuilles en présence et en ce que - à partir d'une commande provenant de la mémoire centrale (7) sur base des données déchiffrées à partir de la mémoire de cassette (3) au poste de lecture (6, 6e), qui concernent le format des cassettes et des feuilles et/ou le type de feuilles - on dépose la feuille (2') retirée de la cassette (1) dans le magasin d'alimentation en feuilles (6g) correspondant à son format et/ou à son type de feuille, à l'intervention d'un moyen de transport connu à cet effet.

13.   Procédé selon la revendication 12, caractérisé en ce qu'on introduit les différents magasins d'alimentation en feuilles (6g) garnis de paquets de feuilles au poste de lecture (6), de manière connue, dans un appareil de chargement de cassettes (11), en ce qu'on positionne une cassette à charger (1) de manière connue dans l'appareil de chargement de cassettes (11), en ce qu'on déchiffre ensuite ces données concernant le format et le type de feuille provenant de sa mémoire de cassette (3) à l'intervention d'un dispositif de lecture de mémoire de cassette et on la transmet à un dispositif de commande de chargement connu en soi à l'intervention duquel le magasin d'alimentation en feuilles correspondant aux données quant au format et au type de feuilles se tient prêt pour le rechargement de la cassette, et en ce qu'on charge enfin la cassette avec la feuille correspondant à ses données de mémoire de cassette, on la ferme et on la retire de l'appareil de chargement (11).

Fig. 1

Röntgenaufnahmegerät

EP 0 307 760 B1

Fig. 2

Röntgen-aufnahme

Fig. 4

Fig. 5

Fig. 3

Fig. 6